# EUROPEAN PATENT APPLICATION

(11) **EP 2 128 161 A1**
(43) Date of publication of application: **02.12.2009**
(21) Application number: 08380165.4
(22) Date of filing: 30.05.2008
(51) Int. Cl.: C07D 495/04, C07D 333/34

(54) **Process for obtaining 4-hydroxy-6-methyl-5,6-dihydro-4H-thieno[2,3-b]thiopyran-7,7-dioxide and its enantiomers, and applications thereof**

(71) Applicant: Ragactives, S.L., 47151 Boecillo (Valladolid) (ES)
(72) Inventor: Silva Guisasola, Luis Octavio Ragactives, S.L.U., 47151 Boecillo-Valladolid (ES); Gorgojo Lobato, Jose Maria, Ragactives,S.L.U., 47151-Boecillo-Valladolid (ES); Gutierrez Fuentes,Luis Gerardo rAGACTIVES, s.l.u, 47151-Boecillo -Valladolid (ES)
(74) Representative: ABG Patentes, S.L.

(57) **Abstract**

The invention relates to a process for obtaining cis-4-hydroxy-6-methyl-5,6-dihydro-4H-thieno[2,3-b]thiopyran-7,7-dioxide, its enantiomers or mixtures thereof, by reduction of 5,6-dihydro-6-methyl-4H-thieno[2,3-b]thiopyran-4-one-7,7-dioxide with a reducing agent which generates hydride ions. The obtained compounds are useful as intermediates in the synthesis of chiral active ingredients.

## Description

### Field of the Invention

The invention generally relates to a process for obtaining 4-hydroxy-6-methyl-5,6-dihydro-4H-thieno[2,3-b]thiopyran-7,7-dioxide and its enantiomers. Said compounds can be used as intermediates in the enantioselective synthesis of pharmaceutical active ingredients. The invention also relates to novel synthesis intermediates generated during the implementation of said process.

### Background of the Invention

One of the current therapies for the control of high intraocular pressure, which seems to be related to the occurrence and progression of glaucoma, consists of the topical administration of a carbonic anhydrase inhibitor. European patent EP 296879 describes some active compounds useful as inhibitors of said carbonic anhydrase, when administered by topical route, such as the compound (4S,6S)-4-(ethylamino)-5,6-dihydro-6-methyl-4H-thieno[2,3-b]thiopyran-2-sulfonamide-7,7-dioxide, the International Nonproprietary Name of which is dorzolamide, of formula:

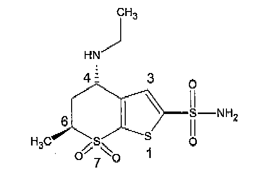

Said European patent EP 296879 describes obtaining dorzolamide by means of a process in which the intermediates used are racemic compounds (enantiomer mixture). Enantiomerically pure dorzolamide requires the use of chiral resolutions in the last step of synthesis, which significantly reduces the global synthesis yield.

Other known synthetic processes for obtaining dorzolamide use, as precursors, intermediates which already have the suitable chirality and which allow achieving the end product in a diastereoselective form. Among said intermediates are the 4-hydroxy-6-alkyl-5,6-dihydro-4H-thieno[2,3-b]thiopyran-7,7-dioxide compounds, which contain two chiral centers in carbons 4 and 6 (hereinafter, chiral hydroxysulfones), of *cis* relative configuration, used as key intermediates in different syntheses.

The transformation of said chiral hydroxysulfones of *cis* configuration into the corresponding 4-ethylamines of *trans* configuration by means of the conversion of the hydroxyl group into a leaving group and subsequent bimolecular nucleophilic substitution with ethylamine (US 5157129), or using as a nucleophile an azide anion which can be subsequently transformed into the ethylamino group (US 5319772), or by means of a Mitsunobu-type reaction with inversion of the configuration (EP 1813618) have been described. The last step for obtaining dorzolamide generally consists of introducing the sulfonamide group in position 2 of the thiophene ring.

Said chiral hydroxysulfones can be obtained by means of a microbiological process (US 5580764 and US 5565345) or by means of chemical synthesis. In relation to this last alternative, United States patent US 5157129 describes a process for obtaining said chiral hydroxysulfone of *cis* configuration which comprises the reduction of the corresponding intermediate [5,6-dihydro-6-alkyl-4H-thieno[2,3-b]thiopyran-4-one-7,7-dioxide] with a borane derivative (e.g., diborane or a borane complex, such as borane-methyl sulfide) as a reducing agent in the presence of an oxazaborolidine. The use of a chiral catalyst (oxazaborolidine) significantly increases the cost of the process and the use of borane derivatives makes the synthesis dangerous.

Therefore, there is a need to develop an alternative synthetic process for obtaining said chiral hydroxysulfones of *cis* configuration that overcomes all or some of the problems associated to the known processes.

### Summary of the Invention

It has surprisingly been found that it is possible to efficiently obtain a chiral hydroxysulfone (specifically, *cis-*4-hydroxy-6-methyl-5,6-dihydro-4H-thieno[2,3-b]thiopyran-7,7-dioxide), its enantiomers and mixtures thereof, by means of the reduction of 5,6-dihydro-6-methyl-4H-thieno[2,3-b]thiopyran-4-one-7,7-dioxide with a suitable reducing agent.

A process such as that provided by this invention has a number of advantages since it not only allows obtaining the desired, virtually pure *cis* diastereoisomer in a simple manner without having to resort to expensive purification techniques (e.g., by chromatography), but it also prevents the use of expensive reagents (e.g., the chiral catalysts used for stereoselectively performing the reduction). This all contributes to reducing the global cost of the process, making the process of the invention an interesting process from a commercial point of view, and it allows being implemented on an industrial level.

Therefore, in one aspect the invention relates to a process for obtaining *cis*-4-hydroxy-6-methyl-5,6-dihydro-4H-thieno[2,3-b]thiopyran-7,7-dioxide, its enantiomers or mixtures thereof, comprising the reduction of 5,6-dihydro-6-methyl-4H-thieno[2,3-b]thiopyran-4-one-7,7-dioxide with a suitable reducing agent.

In another aspect, the invention relates to a process for obtaining *cis*-4-hydroxy-6-methyl-5,6-dihydro-4H-thieno[2,3-b]thiopyran-7,7-dioxide, its enantiomers or mixtures thereof, comprising (i) resolving an enantiomer mixture of 3-(2-thienylthio) butyric acid to obtain the desired enantiomer; (ii) the intramolecular cyclization of said enantiomer; (iii) the oxidation of the ketone obtained and (iv) the reduction of the 5,6-dihydro-6-methyl-4H-thieno[2,3-b]thiopyran-4-one-7,7-dioxide obtained with a reducing agent.

In another aspect, the invention relates to a process for obtaining the desired enantiomer of 3-(2-thienylthio) butyric acid by means of resolving an enantiomeric mixture of 3-(2-thienylthio) butyric acid using an optically active base as a resolution agent.

In another aspect, the invention relates to a process for obtaining (3S)-3-(2-thienylthio)butyric acid by means of chiral resolution of an enantiomeric mixture of 3-(2-thienylthio) butyric acid using an optically active base as a resolution agent.

In another aspect, the invention relates to a diastereoisomeric salt formed by an enantiomer of 3-(2-thienylthio) butyric acid and an optically active base selected from D-(+)-α-methylbenzylamine, 4-nitrophenyl-methylamine, L-lysine, 1-(1-naphthyl)-ethylamine (e.g., (R)-1-(1-naphthyl)-ethylamine), 1-(2-naphthyl)-ethylamine (e.g., (R)-1-(2-naphthyl)-ethylamine), 1-phenylpropylamine and desoxyephedrine. In a particular embodiment, said diastereoisomeric salt is D-(+)-methylbenzylamine (3S)-3-(2-thienylthio)butyrate salt.

In another aspect, the invention relates to a process for the synthesis of (4S,6S)-4-(ethylamino)-5,6-dihydro-6-methyl-4H-thieno[2,3-b]thiopyran-2-sulfonamide-7,7-dioxide (dorzolamide) which comprises performing, at least one of the previously mentioned processes.

### Detailed Description of the Invention

In an aspect, the invention relates to a process, hereinafter process of the invention A, for obtaining *cis*-4-hydroxy-6-methyl-5,6-dihydro-4H-thieno[2,3-b]thiopyran-7,7-dioxide, of formula (I) 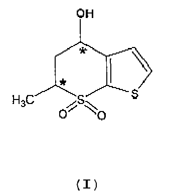 wherein each asterisk (*) indicates an asymmetric carbon atom,
its enantiomers or mixtures thereof,
comprising the reduction of 5,6-dihydro-6-methyl-4H-thieno[2,3-b]thiopyran-4-one-7,7-dioxide of formula (II) 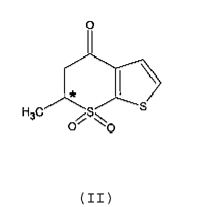 wherein the asterisk (*) has the previously described meaning,
to said compound of formula (I) with a reducing agent which generates hydride ions.

As used herein, a "reducing agent which generates hydride ions" is a compound capable of transferring one or more hydride ions and which are formed or contain one or more electropositive elements selected from groups IA (alkaline metals), IIA (alkaline earth metals) or IIIA, such as for example, lithium, sodium, potassium, aluminum, etc.; in a particular embodiment, the reducing agent which generates hydride ions has two or more different electropositive elements (e.g., lithium and aluminum, etc.); in another particular embodiment, the reducing agent which generates hydride ions further has a non-metallic element such as a non-metallic cation, for example boron (e.g., boron and lithium, boron and sodium, boron and potassium, etc.); and, in another particular embodiment, the reducing agent which generates hydride ions has an anion (e.g., a cyanide group (CN-), etc.). Illustrative, non-limiting examples of reducing agents which generate hydride ions and which can be used as reducing agents in the present invention include sodium borohydride (NaBH₄), potassium borohydride (KBH₄), lithium borohydride (LiBH₄), lithium aluminum hydride (LiAlH₄) or lithium triethylborohydride (LiEt₃BH or lithium super-hydride).

In the compound of formula (I) the prefix *cis* indicates the relative position of the substituents in the ring; specifically, said prefix *cis* indicates that the hydroxyl (position 4) and methyl (position 6) groups are on the same side of a common reference plane formed by the one containing the cyclic backbone. Given that the compound of formula (I) has two chiral centers, one in position 4 and the other in position 6, the absolute stereochemistry (configuration) of said chiral centers is such that the stereochemistry of the substituents in the compound of formula (I) is *cis*; therefore, the compound of formula (I) has 2 enantiomers:
(4R,6S)-4-hydroxy-6-methyl-5,6-dihydro-4H-thieno[2,3-blthiopyran-7,7-dioxide, occasionally identified in this description as (4R,6S)-I; and
(4S,6R)-4-hydroxy-6-methyl-5,6-dihydro-4H-thieno[2,3-b]thiopyran-7,7-dioxide, occasionally identified in this description as (4S,6R)-I.

Said enantiomers are intermediates useful in the synthesis of chemical compounds, including products with therapeutic applications (pharmaceutical active ingredients); by way of illustration, the (4R,6S)-I enantiomer can be used in the synthesis of dorzolamide.

The inventors have surprisingly observed that when a ketone of formula (II) having a methyl group in position 6 is subjected to a reduction reaction with said reducing agent which generates hydride ions, unlike what would be expected, a single enantiomer of the compound of formula (I) is substantially obtained, specifically, the enantiomer in which the hydroxyl group in position 4 is stereoselectively in *cis* position with regard to the methyl group present in position 6. As used herein, "substantially" means that the obtained compound of formula (I) has a *cis*/*trans* ratio typically greater than 90%, preferably greater than 95%, and even more preferably greater than 98%. Therefore, the reduction of the ketone of formula (II) with said reducing agent which generates hydride ions according to the present invention allows obtaining a compound of formula (I), in which the hydroxyl group in position 4 is surprisingly, in an almost completely stereoselective manner, in *cis* position with regard to the methyl group in position 6. Said reduction is surprisingly carried out without the need of having to use (i) expensive chiral catalysts (e.g., oxazaborolidine) to generate a second chiral center with the suitable absolute configuration in position 4 so that the hydroxyl group is in *cis* with regard to the methyl group in position 6, or (ii) borane (boron and hydrogen compound) or derivatives thereof (e.g., diborane or borane-THF or borane-methyl sulfide complexes) as a reducing agent, as described in US 5157129, but using some inexpensive and easy to handle reagents, which significantly reduces the global cost of the process.

As is known, the use of borane or of derivative reagents, such as oxazaborolidines, as reducing agents poses a number of drawbacks since when in contact with water or humidity they can generate hydrogen, which is a volatile, highly inflammable and explosive reagent in contact with air or water; therefore, their high toxicity and explosiveness make them reagents that are dangerous and difficult to handle and store.

According to the process of the invention A, the compound of formula (II) is subjected to a reduction reaction with a reducing agent which generates hydride ions, under conditions allowing the reduction of the keto group to a hydroxyl group, to obtain the compound of formula (I). Although virtually any reducing agent which generates hydride ions can be used to implement the process of the invention, in a particular embodiment said reducing agent is selected from lithium aluminum hydride (LiAlH₄), lithium borohydride (LiEH₄), sodium borohydride (NaBH₄), potassium borohydride (KBH₄), lithium super-hydride (LiEt₃BH) and their mixtures, preferably, NaBH₄.

Said reduction reaction is carried out in a suitable solvent, such as an organic solvent, for example, an aromatic solvent (e.g., toluene, etc.), a halogenated solvent, such as a chlorinated hydrocarbon (e.g., methylene chloride, dichloroethane, etc.), an alcohol, such as a low molecular weight alcohol (e.g., methanol, ethanol, isopropanol, etc.), an ether, for example, an aliphatic ether (e.g., diisopropyl ether, etc.), a cyclic ether (e.g., tetrahydrofuran (THF), methyl-THF, a dioxane, etc.), etc., at a temperature comprised between -75°C and 35°C, for a time period equal to or greater than 15 minutes, typically comprised between 30 minutes and 12 hours, which conditions allow the reduction of the keto group in position 4 to a hydroxyl group for obtaining the compound of general formula (I).

In a particular embodiment, a compound of formula (I), in which the hydroxyl group in position 4 is in *cis* with regard to the methyl group in position 6, is obtained by means of reduction of the keto group present in position 4 of the compound of formula (II) using NaBH₄ as a reducing agent and methanol as a solvent, at a reaction temperature comprised between -5°C and 20°C.

The compound of formula (II) has only one chiral center in position 6, whereas the reduced compound of formula (I) has an additional chiral center in position 4. Therefore, depending on the absolute stereochemistry of the initial chiral center present in the compound of formula (II), two enantiomers of the compound of formula (I) maintaining the *cis* relative stereochemistry between the hydroxyl and methyl groups in positions 4 and 6 respectively, specifically, the (4R,6S)-I and [4S,6R)-I enantiomers, can be obtained. In fact, when the compound of formula (II) is a single enantiomer, the obtained compound of formula (I) is also a single enantiomer, specifically, that in which the absolute configuration of the chiral centers (positions 4 and 6) is such that the hydroxyl group in position 4 is in *cis* with regard to the methyl group in position 6.

Therefore, in a particular embodiment, the compound of formula (II) is a single enantiomer in which the methyl group in position 6 has (S) configuration and the obtained compound of formula (I) is the enantiomer in which the new chiral center formed in position 4 has (R) configuration and the hydroxyl group in position 4 is in *cis* with regard to the methyl group in position 6, specifically, the enantiomer (4R,6S)-I.

In another particular embodiment, the compound of formula (II) is a single enantiomer in which the methyl group in position 6 has (R) configuration and the obtained compound of formula (I) is the enantiomer in which the new chiral center formed in position 4 has (S) configuration and the hydroxyl group in position 4 is in *cis* with regard to the methyl group in position 6, specifically the (45,6R)-I enantiomer.

In another particular embodiment, the compound of formula (II) is an [(R) and (S)] enantiomer mixture and the obtained compound of formula (I) is an enantiomer mixture in which the hydroxyl group in position 4 is in *cis* with regard to the methyl group in position 6, specifically a (4R,6S)-I and (4S,6R)-I enantiomer mixture. As used in this description, the term "enantiomer mixture" or "enantiomeric mixture" refers to any enantiomer mixture of a compound independently of the relative proportion between them; i.e., said mixtures can contain an excess of one of the enantiomers with regard to the other or can contain equimolecular amounts of said enantiomers, in which case they would be racemic mixtures.

In a particular embodiment, the compound of formula (II) is selected from (6S)-5,6-dihydro-6-methyl-4H-thieno[2,3-b]thiopyran-4-one-7,7-dioxide and (6R)-5,6-dihydro-6-methyl-4H-thieno[2,3-b]thiopyran-4-one-7,7-dioxide and the obtained compound of formula (I) is (4R,6S)-4-hydroxy-6-methyl-5,6-dihydro-4H-thieno[2,3-b]thiopyran-7,7-dioxide [(4R,6S)-I] or (4S,6R)-4-hydroxy-6-methyl-5,6-dihydro-4H-thieno[2,3-b]thiopyran-7,7-dioxide [(4S,6R)-I], respectively.

In another aspect, the invention relates to a process, hereinafter process of the invention B, for obtaining *cis*-4-hydroxy-6-methyl-5,6-dihydro-4H-thieao[2,3-b]thiopyran-7,7-dioxide of formula (I) 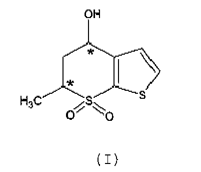 wherein each asterisk (*) indicates an asymmetric carbon atom,
its enantiomers or mixtures thereof,
comprising
a) resolving an enantiomer mixture of 3-(2-thienylthio) butyric acid to obtain the desired enantiomer of formula (III); 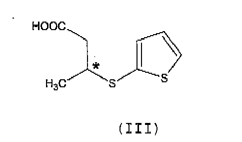 wherein the asterisk (*) has the previously indicated meaning;
b) subjecting said enantiomer of formula (III) to an intramolecular cyclization reaction to obtain the compound 5,6-dihydro-6-methyl-4H-thieno[2,3-b]thiopyran-4-one of formula (IV) 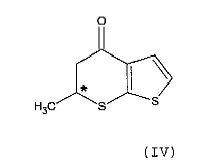 wherein the asterisk (*) has the previously indicated meaning;
c) converting said compound of formula (IV), by means of an oxidation reaction, into a compound of formula (II) 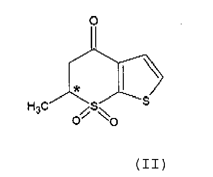 wherein the asterisk (*) has the previously indicated meaning; and
d) subjecting said compound of formula (II) to a reduction reaction, by means of the reaction of said compound of formula (II) with a reducing agent which generates hydride ions, to obtain the compound of formula (I).

3-(2-thienylthio) butyric acid is a known product that has a chiral center. Obtaining 3-(2-thienylthio) butyric acid in its racemic form has been described in several documents (e.g., US 4968814, US 4968815, US 5723628 and US 5756768).

In its step a), the process of the invention B comprises obtaining the desired enantiomer of 3-(2-thienylthio) butyric acid by means of resolving an enantiomer mixture of said 3-(2-thienylthio) butyric acid with a suitable resolution agent, such as an (optically active base. Said enantiomer mixture can be a racemic mixture or a mixture enriched with any one of enantiomers. Obtaining said desired enantiomer of 3-(2-, thienylthio) butyric acid of formula (III) by means of fractional crystallization of the corresponding diastereoisomeric salts formed by the reaction of enantiomers of 3-(2-thienylthio) butyric acid with said suitable resolution agent and subsequent release of the desired enantiomer is an additional aspect of the present invention and will be described in detail below, when describing the "resolution process of the invention".

In a particular embodiment, the desired enantiomer of formula (III) is selected from (3S)-3-(2-thienylthio) butyric acid and (3R)-3-(2-thienylthio) butyric acid, preferably (35)-3-(2-thienylthio) butyric acid.

In step b), the desired enantiomer of the compound of formula (III) is subjected to an intramolecular cyclization reaction to obtain the compound of general formula (V) by conventional methods, for example, by means of any of the already previously described experimental conditions, such as the use of (CO)₂Cl₂/SnCl₄ (EP 296879) or by means of trifluoroacetic anhydride (EP 453288).

In step c), the compound of formula (II) can be obtained by means of oxidation of the sulfur in position 7 of the compound of formula (V) by conventional methods, for example, by means of the use of already described experimental conditions, for example, by means of the use of Na₂WO₄/H₂O₂, as described in EP 453288 A1 or in several publications, G. Sosnovsky et al., Z. Naturforsch. 31b, 1376 (1976); and E. G. Rozantsev et al., Synthesis, 1971, 190.

In step d), the compound of formula (I) is obtained by subjecting the compound of formula (II) to a reduction reaction with a reducing agent which generates hydride ions, under conditions that allow the reduction of the keto group to a hydroxyl group, to obtain the compound of formula (I). Illustrative, non-limiting examples of said reducing agent which generates hydride ions include lithium aluminum hydride (LiAlH₄), lithium borohydride (LiBH₄), sodium borohydride (NaBH₄), potassium borohydride (KBH₄), lithium super-hydride (LiEt₃BH) and their mixtures, preferably NaBH₄. Said reaction is carried out in a suitable solvent, such as an organic solvent, for example, an aromatic solvent (e.g., toluene, etc.), an halogenated solvent, such as a chlorinated hydrocarbon (e.g., methylene chloride, dichloroethane, etc.), an alcohol, such as a low molecular weight alcohol (e.g., methanol, ethanol, isopropanol, etc.), an ether, for example, an aliphatic ether (e.g., diisopropyl ether, etc.), a cyclic ether (e.g., tetrahydrofuran (THF), methyl-THF, a dioxane, etc.), etc., at a temperature comprised between -75°C and 35°C, for a time period equal to or greater than 15 minutes, typically comprised between 30 minutes and 12 hours, which conditions allow the reduction of the keto group in position 4 to a hydroxyl group to obtain the compound of general formula (I).

In a particular embodiment, if the compound (II) is a single enantiomer, a compound of formula (I) with a *cis*-(I) enantiomer yield greater than 95%, typically greater than 98%, can be obtained.

In another particular embodiment, a compound of formula (I), in which the hydroxyl group in position 4 is in *cis* with regard to the methyl group in position 6, is obtained by means of reduction of the keto group present in position 4 of the compound of formula (II) using NaBH₄ as a reducing agent which generates hydride ions and methanol as a solvent, at a reaction temperature comprised between -5°C and 20°C.

In another particular embodiment, a compound of formula (I), in which the hydroxyl group in position 4 is in *cis* with regard to the methyl group in position 6, is obtained by means of reduction of the keto group present in position 4 of the compound of formula (II) using LiAlH₄ as a reducing agent and THF as a solvent, at a reaction temperature comprised between - 5°C and 20°C.

In another particular embodiment, a compound of formula (I), in which the hydroxyl group in position 4 is in *cis* with regard to the methyl group in position 6, is obtained by means of reduction of the keto group present in position 4 of the compound of formula (II) using LiEt₃BH as a reducing agent.

As previously mentioned in relation to step a) of the process of the invention B, the resolution of the enantiomeric mixture of 3-(2-thienylthio) butyric acid to obtain the desired enantiomer of formula (III) is carried out by means of an original process and is an additional aspect of this invention.

Therefore, in another aspect, the invention relates to a process for obtaining the desired enantiomer of 3-(2-thienylthio) butyric acid by means of resolving the enantiomeric mixture of 3-(2-thienylthio) butyric acid, hereinafter resolution process of the invention, comprising:
a) forming diastereoisomeric salts by means of reaction of the enantiomers of said enantiomeric mixture with a resolution agent, wherein said resolution agent is an optically active base;
b) recrystallizing the diastereoisomeric salts obtained in step a) in a solvent to obtain a diastereoisomeric salt enriched with the desired enantiomer; and
c) obtaining the desired enantiomer by means of:
   c.1) resuspending the diastereoisomeric salt obtained in step b) in a suitable medium and rupture of the salt in basic medium.
   c.2) neutralizing the separated acid by means of its acidification until achieving a pH comprised between 5 and 1; and
   c.3) isolating the desired enantiomer of 3-(2-thienylthio) butyric acid.

The enantiomeric mixture of 3-(2-thienylthio) butyric acid can be a racemic mixture or a mixture enriched with any one of the enantiomers.

Now it has surprisingly been found that the desired enantiomer of 3-(2-thienylthio) butyric acid can be obtained by means of fractional crystallization of the corresponding diastereoisomeric salts formed by the reaction of enantiomers of 3-(2-thienylthio) butyric acid with a suitable resolution agent, such as an optically active base. Said optically active base useful as a resolution agent can be any optically active base which forms diastereoisomeric salts with the enantiomers of 3-(2-thienylthio) butyric acid and allows the separation of the desired enantiomer. Although virtually any optically active base which meets said conditions can be used, in a particular embodiment, said optically active base is selected from D-(+)-α-methylbenzylamine, 4-nitrophenyl-methylamine, L-lysine, 1-(1-naphthyl)-ethylamine (e.g., (R)-1-(1-naphthyl)-efihylamine), 1-(2-naphthyl)-ethylamine (e.g., (R)-1-(2-naphthyl)-ethylamine), 1-phenylpropylamine and desoxyephedrine, preferably D-(+)-α-mefihylbenzylamine. The diastereoisomeric salts formed by the reaction of enantiomers of 3-(2-thienylthio) butyric acid with said optically active bases are crystalline, therefore, when subjected to a crystallization process in a suitable solvent, one of said diastereoisomeric salts can be separated as a main product, and, subsequently, the desired enantiomer of 3-(2-thienylthio) butyric acid can be recovered.

Briefly, in step a), the enantiomer mixture of 3-(2-thieriylthio) butyric acid comes into contact with the resolution agent (optically active base) under conditions that allow the formation of the corresponding diastereoisomeric salts, and, if desired, their isolation by conventional methods, for example, by precipitation. The reaction of enantiomers of 3-(2-thienylthio) butyric acid with the optically active base is carried out in a suitable crystallization solvent (or mixture of solvents), such as a solvent or mixture of solvents which allows completely or partially dissolving the diastereoisomeric salts and their subsequent precipitation for the purpose of obtaining the diastereoisomeric salt enriched with the desired enantiomer of 3-(2-thienylthio) butyric acid. In a particular embodiment, said crystallization solvent is an organic solvent, optionally mixed with water; illustrative, non-limiting examples of organic solvents that can be used as crystallization solvents include alcohols, such as low molecular weight alcohols (e.g., methanol, ethanol, isopropanol, etc.), ethers (e.g., tetrahydrofuran, methyl THF, etc.), esters (e.g., ethyl acetate, etc), ketones (e.g., acetone, etc.), nitriles (e.g., acetonitrile, etc.), etc., their mixtures, or their mixtures with water. In a specific embodiment, said crystallization solvent is acetonitrile, optionally mixed with water.

The resolution agent (optically active base) can be used in a variable proportion with regard to the enantiomer mixture of 3-(2-thienylthio) butyric acid to be separated, for example, in a ratio of 0.4 to 1.5 equivalents of resolution agent to equivalent of enantiomer present in the enantiomer mixture. In a particular embodiment, said ratio of equivalents of resolution agent to equivalent of enantiomer present in the enantiomer mixture is from 0.5 to 0.7 equivalents.

In step b) the diastereoisomeric salts obtained in step a) are recrystallized in a suitable solvent to obtain a diastereoisomeric salt enriched with the desired enantiomer. The recrystallization of the previously obtained diastereoisomeric salts is carried out by conventional methods, for example, heating the mixture of the diastereoisomeric salts and the crystallization solvent (e.g., until boiling) and then cooling, controlling the variables of the crystallization process (e.g., cooling rate, crystallization temperature and crystallization time), which will depend on the nature of the diastereoisomeric salt to be separated and can be routinely established by a person skilled in the art; in an illustrative, non-limiting manner, the cooling rate can be comprised within a wide range, such as between 0.5°C/min and 5°C/min, for example, 1°C/min, 2°C/min, 4°C/min, etc.; the crystallization temperature can vary within a wide range, for example, between 0°C and 20°C, between 20°C and 40°C, between 40°C and 60°C, etc.; likewise, the crystallization time can vary within a wide range, for example, between 0.5 and 3 hours, between 4 and 6 hours, between 6 and 10 hours, etc.

In a particular embodiment, the resolution agent is D-(+)-methylbenzylamine; the crystallization solvent is acetonitrile, the diastereoisomeric salts formed are D-(+)-methylbenzylamine (3S)-3-(2-thienylthio)butyrate salt and D-(+)-methylbenzylamine (3R)-3-(2-thienylthio) butyrate salt which are separated by slowly cooling the reaction mixture to a temperature of 20-25°C for a time period comprised between 8 and 10 hours. Said diastereoisomeric D-(+)-methylbenzylamine (3S)-3-(2-thienylthio)butyrate and D-(+)-methylbenzylamine (3R)-3-(2-thienylthio)butyrate salts are additional aspects of this invention.

The desired enantiomer of 3-(2-thienylthio) butyric acid can be obtained from the corresponding diastereoisomeric salt by conventional methods, for example, by means of neutralization, as mentioned in step c). Briefly, by way of a non-limiting illustration, the diastereoisomeric salt comprising the desired enantiomer of the previously obtained 3-(2-thienylthio) butyric acid is dissolved or resuspended in a suitable solvent [step c.1)], such as an aqueous organic medium comprising water, and at least one suitable organic solvent, such as a halogenated solvent (e.g., methylene chloride, etc.), an aromatic solvent (e.g., toluene, etc.), an ether (e.g., THF, methyl-THF, etc.), etc., and the pH is adjusted to a basic pH, for example between 10 and 12, to release the 3-(2-thienylthio) butyric acid from the resolution agent by means of the formation of another preferably inorganic salt; then, after the phase separation, the aqueous phase which contains the non-diastereoisomeric salt of 3-(2-thienylthio) butyric acid, free of the optically active base (chiral amine), is acidified adding an acid until achieving a pH comprised between 5 and 1 [step c.2)], preferably, between 4 and 2, whereby the desired enantiomer of 3-(2-thienylthio) butyric acid is neutralized. Virtually any suitable strong, organic or inorganic acid can be used for said neutralization; nevertheless, in a particular embodiment, said acid is selected from the group formed by hydrochloric acid, hydrobromic acid, sulfuric acid, nitric acid, etc., and their mixtures. Subsequently, the desired enantiomer of 3-(2-thienylthio) butyric acid, which normally will be in the organic phase, is isolated [step c.3)] by conventional methods, in the form of a free acid (e.g., as an oil, etc.) or in the form of a salt by means of the formation of a salt with a suitable base, for example, triethylamine, sodium hydroxide, etc.

In a particular embodiment, the separation of the (S) enantiomer of 3-(2-thienylthio) butyric acid [(3S)-3-(2-thienylthio) butyric acid] is performed by means of optical resolution by reacting the enantiomer mixture of 3-(2-thienylthio) butyric acid with D-(+)-a-methylbenzylamine (resolution agent). The diastereoisomeric salt containing the desired enantiomer can be recrystallized the necessary times until obtaining the desired enantiomer of 3-(2-thienylthio) butyric acid, e.g., (3S)-3-(2-thienylthio) butyric acid, with the desired purity.

Said diastereoisomeric salts, obtained by reaction of enantiomers of 3-(2-thienylthio) butyric acid and said resolution agent, as well as their crystallization and release to obtain the desired enantiomer of 3-(2-thienylthio) butyric acid [compound of formula (III)] have not been previously described and involve an advantageous method for obtaining said enantiomers of formula (III) with the desired stereochemistry and are an additional aspect of the present invention. In a particular embodiment, said diastereoisomeric salt is selected from D-(+)-methylbenzylamine (3S)-3-(2-thienylthio)butyrate salt and D-(+)-methylbenzylamine (3R)-3-(2-thienylthio) butyrate salt.

In a particular embodiment, the invention provides a process for obtaining (3S)-3-(2-thienylthio) butyric acid by means of the chiral resolution of an enantiomeric mixture of 3-(2-thienylthio) butyric acid, using an optically active base as a resolution agent, in a medium comprising an organic solvent or an aqueous organic mixture as a solvent, in which the diastereoisomeric salts formed are subjected to one or more recrystallizations followed by isolation and recovery of the desired enantiomer [(3S)-3-(2-thienylthio) butyric acid] by rupture of the diastereoisomeric salt by means of basification in aqueous organic medium, phase separation and subsequent neutralization (acidulation) of the aqueous phase in the presence of an organic phase with an acid medium and subsequent phase separation.

The compound of formula (I) can be used as an intermediate in the synthesis of dorzolamide [(4S,6S)-4-(ethylamino)-5,6-dihydro-6-methyl-4H-thieno[2,3-b]thiopyran-2-sulfonamide-7,7-dioxide] by means of conventional methods, for example, by means of the transformation of said compound of formula (I), particularly of the compound (4R,6S)-I, into the corresponding *trans* [(4S,6S)-4-(ethylamino)-5,6-dihydro-6-methyl-4H-thieno[2,3-b]thiopyran-7,7-dioxide] ethylamine by means of the conversion of the hydroxyl group into a leaving group and subsequent bimolecular nucleophilic substitution with ethylamine, as described in US 5157129, and subsequent introduction of the sulfonamide group in position 2 of the thiophene ring. Alternatively, said transformation can be performed using as a nucleophile an azide anion that is subsequently transformed into the ethylamino group (US 5319772). Likewise, in another alternative embodiment, said transformation of the hydroxyl group in position 4 of the compound (I) into the corresponding ethylamino group in *trans* with regard to the methyl group in position 6 is performed by means of a Mitsunobu reaction with inversion of the configuration (EP 1813618) and subsequent introduction of the sulfonamide group in position 2 of the thiophene ring. The introduction of said sulfonamide group in said position can be performed by directly reacting said compound (4S,6S)-4-(ethylamino)-5,6-dihydro-6-methyl-4H-thieno[2,3-b]thiopyran-7,7-dioxide with fuming sulfuric acid (US 5157129), or prior protection of the amino group by means of the formation of an amide and subsequent addition of chlorosulfonic acid, chlorination of the resulting intermediate by means of the addition of thionyl chloride, and the formation of the sulfonamide by the addition of aqueous ammonia (US 7030250). As can be seen, the compound of formula (I) and the previously described processes of the invention are especially useful in the synthesis of dorzolamide. Therefore, an additional aspect of this invention is the use of one of the processes of the invention (A or B) in the synthesis of dorzolamide, as well as a process for the synthesis of dorzolamide comprising the use of (3S)-3-(2-thienylthio) butyric acid obtained by resolving 3-(2-thienylthio) butyric acid with an optically active base (e.g., D-(+)-methylbenzylamine, 4-nitrophenyl-methylamine, L-lysine, 1-(1-naphthyl)-ethylamine (e.g., (R)-1-(2-naphthyl)-ethylamine), 1-(2-naphthyl)-ethylamine (e.g., (R)-1-(2-naphthyl)-ethylamine), 1-phenylpropylamine or desoxyephedrine) as a resolution agent, or the use of the diastereoisomeric salts formed by reaction of 3-(2-thienylthio) butyric acid with said optically active base, as has been previously described.

In a particular embodiment, the process for obtaining dorzolamide comprises a reduction step for reducing the compound 5,6-dihydro-6-methyl-4H-thieno[2,3-b]thiopyran-4-one-7,7-dioxide with a reducing agent which generates hydride ions to obtain the intermediate *cis*-4-hydroxy-6-methyl-5,6-dihydro-4H-thieno[2,3-b]thiopyran-7,7-dioxide, its enantiomers or mixtures thereof (process of the invention A).

In another particular embodiment, the process for obtaining dorzolamide comprises performing the steps of: (i) resolution of an enantiomer mixture of 3-(2-thienylthio) butyric acid to obtain the desired enantiomer; (ii) intramolecular cyclization of said enantiomer; (iii) oxidation of the obtained ketone; and (iv) reduction of the 5,6-dihydro-6-methyl-4H-thieno[2,3-b]thiopyran-4-one-7,7-dioxide obtained with a reducing agent which generates hydride ions, to obtain the compound *cis*-4-hydroxy-6-methyl-5,6-dihydro-4H-thieno[2,3-b]thiopyran-7,7-dioxide, its enantiomers or mixtures thereof (process of the invention B).

The following examples illustrate the invention and must not be considered as limiting of the scope of the same.

### EXAMPLE 1

### Obtaining the D-(+)-methylbenzylamine (3S)-3-(2-thienylthio)butyrate salt

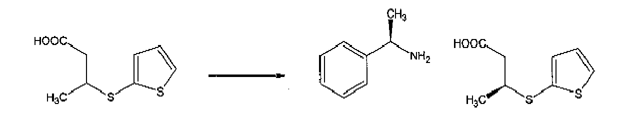

77.19 g (0.38 moles) of racemic 3-(2-thienylthio) butyric acid were dissolved in 1,310 mL of acetonitrile and were heated until the reflux temperature, then 28.1 mL (0.22 moles) of D-(+)-methylbenzylamine were added. The reaction mixture was slowly cooled to a temperature of 20-25°C for a time period comprised between 8 and 10 hours, and was kept under stirring at this temperature for 4-5 hours. The obtained solid was filtered by washing the cake with 77 mL of acetonitrile. The solid thus obtained was suspended again in acetonitrile (679 mL) and was heated until reflux temperature. The cooling and filtering process were repeated 2 more times, a solid finally being obtained which, after being dried, provided 43.36 g (0.14 moles) of the compound of the title with a molar yield of 35% and an enantiomeric excess of 99.1%.

Melting point (Mp): 137.1-138.5°C
¹H NMR (CDCl₃): δ 8.05 (1H, bs), 7.36-7.22 (6H, m), 7.06 (1H, dd), 6.97 (1H, m), 4.19 (1H, m), 3.06 (1H, m), 2.24 (1H, dd), 1.91 (1H, dd), 1.48 (3H, d), 1.06 (3H, d).

### EXAMPLE 2

### Obtaining (3S)-3-(2-thienylthio) butyric acid

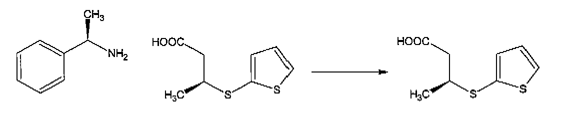

22.07 g (0.07 moles) of the resolved D-(+)-methylbenzylamine (3S)-3-(2-thienylthio)butyrate salt were added to 84 mL of toluene and 84 mL of water, the pH of the dissolution was adjusted to 11-12 with an aqueous solution of NaOH, the mixture was kept under stirring until achieving complete dissolution and the obtained phases were separated. The aqueous phase was mixed with 84 mL of toluene and kept under stirring, the pH was adjusted to 1-2 with an aqueous solution of concentrated HCl, and the phases were separated. The organic phase was concentrated to a residue, 13.17 g of the compound of the title in the form of oil being obtained.

The enantiomeric excess is measured by means of the transformation of (3S)-3-(2-thienylthio) butyric acid into a diastereoisomeric salt mixture by HPLC (High Performance Liquid Chromatography); these salts are obtained by means of dissolving the obtained oil in toluene by adding D-(+)-methylbenzylamine and EEDQ (N-ethoxycarbonyl-2-ethoxy-1,2-dihydroquinoline), heating to reflux, cooling, distilling the toluene and dissolving in acetonitrile. The enantiomeric excess is 99%.

¹H NMR (CDCl₃): δ 11.08 (1H, bs), 7.40 (1H, dm), 7.17 (1H, dm), 7.03 (1H, m), 3.35 (1H, m), 2.65 (1H, dd), 2.41 (1H, dd), 1.32 (3H, d)

### EXAMPLE 3

### Obtaining (6S)-5,6-dihydro-6-methyl-4H-thieno[2,3-b]thiopyran-4-one

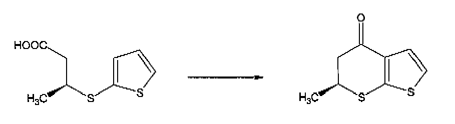

13.14 g (0.06 moles) of (3S)-3-(2-thienylthio) butyric acid were dissolved in 30 mL of toluene at room temperature (18-22°C); the resultant solution was cooled at a temperature between 0-5°C and 5.20 mL (0.07 moles) of trifluoroacetic anhydride (TFAA) were added, keeping the temperature at 0-5°C for one hour, then allowing the temperature to increase to 20-25°C until the end of the reaction. Subsequently, 26 mL of water were slowly added without surpassing 10°C, it was stirred and the phases were decanted. The organic phase was washed with 26 mL of water 2 times, neutralized until pH 6-8 and distilled until achieving 3.5 volumes, being directly used in the following reaction.

Alternatively, the product can be isolated by precipitation in toluene.
Mp: 43.4-44.8°C
¹³C NMR (CDCl₃) δ: 190, 152, 135, 126, 122, 47, 41, 20
¹H NMR (CDCl₃) δ: 7.40 (1H, d), 6.97 (1H, d), 3.76 (1H, m), 2.84 (1H, ddd), 2.64 (1H, ddd), 1.44 (3H, d)
α _{D}: -91.02 c: 1 in CH₂Cl₂

### EXAMPLE 4

### obtaining (6S),-5,6-dihydro-6-methyl-4H-thieno[2,3-b]thiopyran-4-one-7,7-dioxide

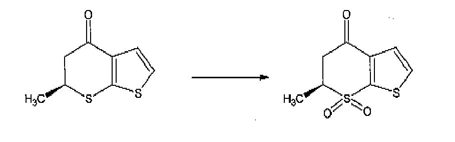

10.22 g (0.055 moles) of (6S)-5.6-dihydro-6-methyl-4H-thieno[2,3-b]thiopyran-4-one were dissolved in 30 mL of toluene and 46 mL of ethyl acetate; it then was cooled to a temperature of 0-5°C and 2.65 g (0.008 moles) of Na₂WO₄·2H₂O, 1.5 mL of water were loaded.

Then, 19.8 mL (0.19 moles) of 30% H₂O₂ were added, keeping the temperature below 10°C and it was stirred until finishing the starting product. The phases were decanted and the organic phase was washed with an aqueous solution of NaCl. The aqueous phase was extracted with ethyl acetate (AcOEt) and the obtained organic phases were pooled together. The organic phase was distilled to a residue, 13 mL of methanol were added and the suspension was cooled at a temperature of 0-5°C, the obtained solid was filtered and dried, 8.5 g of a white solid being obtained, which corresponds to the compound of the title with an 80% yield.
Mp: 97.5-98.2°C
¹³C NMR (CDCl₃) δ: 187, 147, 141, 131, 127, 58, 45, 12
¹H NMR (CDCl₃) δ: 7.58 (1H, d), 7.46 (1H, d), 3.84 (1H, m), 3.18 (2H, m), 1.55 (3H, d)
α _{D}: -7.04 c: 1 in CH₂Cl₂

### EXAMPLE 5

### Obtaining (6S)-5,6-dihydro-6-methyl-4H-thieno[2,3-b]thiopyran-4-one-7,7-dioxide.

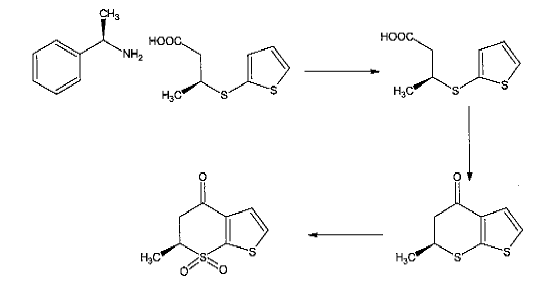

42.13 g (0.13 moles) of the resolved D-(+)-methylbenzylamine (35)-3-(2-thienylthio)butyrate salt were added to 168 mL of toluene and 168 mL of water. The pH was adjusted to 11-12 with an aqueous solution of NaOH and the mixture was kept under stirring until total dissolution, then separating the obtained phases. The aqueous phase was mixed with 168 mL of toluene, the pH was adjusted to 2-4 with an aqueous dissolution of concentrated HCl and the phases were separated. The organic phase was concentrated until achieving 1.5 volumes against resolved salt and was cooled at 0-5°C; 18.96 mL (0.13 moles) of trifluoroacetic anhydride (TFAA) were added to this salt, keeping the temperature for one hour at 0-5 °C and allowing it to increase to room temperature until the end of the reaction. It was subsequently cooled again with water/ice and 53 mL of water were slowly added without surpassing 10°C. It was stirred and the phases were decanted; the organic phase was washed with 53 mL of water two times. The organic phase was diluted with 92 mL of ethyl acetate (AcOEt) and 5.9 g (0.018 moles) of Na₂WO₄·2H₂O and 3.0 mL of water were added and it was cooled to 0-5°C.

39.6 mL (0.38 moles) of 30% H₂O₂ were then slowly added, keeping the temperature below 10°C and it was left under stirring until finishing the starting product. The phases were decanted and the organic phase was washed with an aqueous solution of NaCl. The aqueous phase was extracted with AcOEt and the obtained organic phases were pooled together. The organic phase was distilled to a residue, 26 mL of methanol were added and the obtained suspension was cooled at 0-5°C and was filtered washing with a minimum amount of MeOH. The obtained wet cake was dried, 18 g of a white solid, corresponding to the compound of the title being obtained with 64% accumulated yield.

### EXAMPLE 6

### Obtaining (4R,6S)-4-hydroxy-6-methyl-5,6-dihydro-4H-thieno[2,3-b]thiopyran-7,7-dioxide

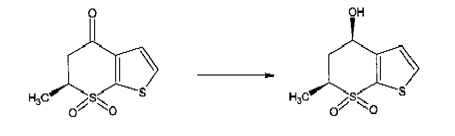

A suspension of 23.81 g (0.11 moles) of (6S)-5,6-dihydro-6-methyl-4H-thieno[2,3-b]thiopyran-4-one-7,7-dioxide was prepared in 375 mL of methanol and was cooled at 0-5°C, then, and 1.25 g (0.3 equivalents) of NaBH₄ were then added in portions without surpassing 5°C. Once the reaction has finished, 140 mL of water were added, the MeOH was eliminated by reduced pressure distillation and the pH was adjusted to 6.5-7 with acetic acid, 500 mL of CH₂Cl₂ were subsequently added, the reaction mixture was stirred and the phases were separated; the aqueous phase was extracted again with 140 mL of CH₂Cl₂. The organic phases were pooled together and the solvent was eliminated by reduced pressure distillation. 30 mL of MeOH were added to the resulting residue and the resulting suspension was cooled and filtered to give 17.4 g of the compound of the title with a yield of 71%.
Mp: 147.2-149.1°C
¹³C NMR (C_{D}Cl₃) δ: 146, 136, 131, 127, 66, 57, 40, 12
¹H NMR (CDCl₃) δ: 7.56 (1H, d), 7.14 (1H, d), 4.88 (1H, m), 3.44 (1H, m), 2.38 (1H, m), 1.62 (1H, bs), 1.50 (3H, d)
α _{D}: -81.82 c: 1 in CH₂Cl₂

### EXAMPLE 7

### obtaining (4R,65)-4-hydroxy-6-methyl-5,6-dihydro-4H-thieno[2,3-b]thiopyran-7,7-dioxide

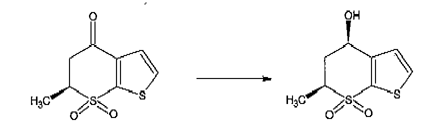

A suspension with 1.0 Kg of (6S)-5,6-dihydro-6-methyl-4H-thieno[2,3-b]thiopyran-4-one-7,7-dioxide was prepared in 15.76 L of methanol and was cooled at 0-5°C. Then, 52 g of NaBH₄ were added, in portions, without surpassing 10°C. Once the reaction has finished, 5.88 L of water were added. It was distilled at a reduced pressure to eliminate the methanol and the pH was adjusted to 6.5-7 with acetic acid. Then 25 L of CH₂Cl₂ were added, it was stirred and the phases were separated. The aqueous phase was extracted with 6 L of CH₂Cl₂. The organic phases were pooled together and distilled to a minimum volume. 3 L of toluene were added, distilled to a minimum volume; it was cooled and filtered, 0.85 Kg of the compound of the title being obtained with a yield of 84%.

### EXAMPLE 8

### Obtaining (4R,6S)-4-hydroxy-6-methyl-5,S-dihydro-4H-thieno[2,3-b]thiopyran-7,7-dioxide

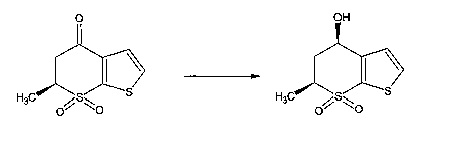

A suspension of (6S)-5,6-dihydro-6-methyl-4H-thieno[2,3-b]thiopyran-4-one-7,7-dioxide was prepared in THE and was cooled at 0-5°C, then 1.0 equivalents of LiAlH₄ were added in portions without surpassing 5°C. Once the reaction has finished, it is cooled again at 0-5°C; a solution of 10% NaOH in water is added, insolubles were filtered and THF was eliminated by reduced pressure distillation and the pH was adjusted to 6.5-7 with acetic acid, subsequently CH₂Cl₂ is added, and the process described in Example 6 is followed.

### EXAMPLE 9

### Obtaining (4R,6S)-4-hydroxy-6-methyl-5,6-dihydro-4H-thieno[2,3-b]thiopyran-7,7-dioxide

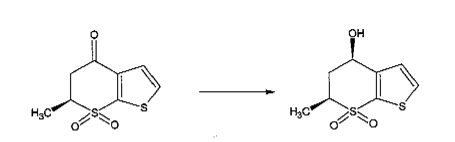

A solution prepared with (6S)-5,6-dihydro-6-methyl-4H-thieno[2,3-b]thiopyran-4-one-7,7-dioxide in Toluene is cooled at -50°C; a solution of 1 M LiEt₃BH in THF without surpassing the temperature of - 40°C is slowly added. Subsequently, once the reaction is completed the temperature is increased until achieving 0°C and water is slowly added without the temperature surpassing 5°C. Phases are separated and the process described in Example 6 is followed.

## Claims

1. A process for obtaining *cis*-4-hydroxy-6-methyl-5,6-dihydro-4H-thieno[2,3-b]thiopyran-7,7-dioxide of formula (I). 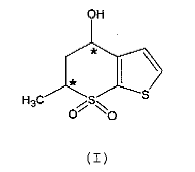 wherein each asterisk (*) indicates an asymmetric carbon atom,
its enantiomers or mixtures thereof,
comprising the reduction of 5,6-dihydro-6-methyl-4H-thieno[2,3-b]thiopyran-4-one-7,7-dioxide of formula (II) 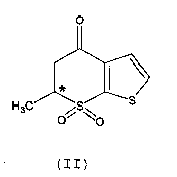 wherein the asterisk has the previously described meaning, to said compound of formula (I) with a reducing agent which generates hydride ions.

2. The process according to claim 1, wherein the obtained compound of formula (I) is selected from (4R,6S)-4-hydroxy-6-methyl-5,6-dihydxo-4H-thieno[2,3-b]thiopyran-7,7-dioxide and (4S,6R)-4-hydroxy-6-methyl-5,6-dihydro-4H-thieno[2,3-b]thiopyran-7,7-dioxide.

3. The process according to claim 1, wherein said reducing agent which generates hydride ions is selected from lithium aluminum hydride (LiAlH₄), lithium borohydride (LiBH₄), sodium borohydride (NaBH₄), potassium borohydride (KBH₄), lithium super-hydride (LiEt₃BH) and their mixtures.

4. A process for obtaining *cis*-4-hydroxy-6-methyl-5,6-dihydro-4H-thieno[2,3-b]thiopyran-7,7-dioxide of formula (I) 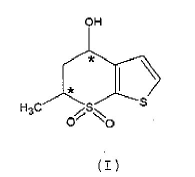 wherein each asterisk (*) indicates an asymmetric carbon atom,
its enantiomers or mixtures thereof,
comprising
a) resolving an enantiomer mixture of 3-(2-thienylthio) butyric acid to obtain the desired enantiomer of formula (III); 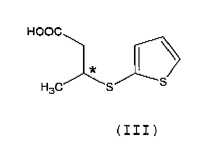 wherein the asterisk (*) has the previously indicated meaning;
b) subjecting said enantiomer of formula (III) to an intramolecular cyclization reaction to obtain the compound 5,6-dihydro-6-methyl-4H-thieno[2,3-b]thiopyran-4-one of formula (IV) 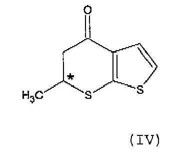 wherein the asterisk (*) has the previously indicated meaning;
c) converting said compound of formula (IV), by means of an oxidation reaction, into a compound of formula (II) 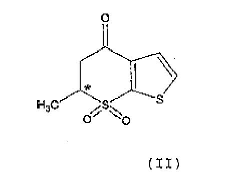 wherein the asterisk (*) has the previously indicated meaning; and
d) subjecting said compound of formula (II) to a reduction reaction, by means of the reaction of said compound of formula (II) with a reducing agent which generates hydride ions, to obtain the compound of formula (I).

5. The process according to claim 4, wherein resolution step a) for resolving the desired enantiomer is performed by means of fractional crystallization of diastereoisomeric salts formed from the reaction of enantiomers with an optically active base.

6. The process according to claim 5, wherein said optically active base is selected from D-(+)-α-methylbenzylamine, 4-nitrophenyl-methylamine, L-lysine, 1-(1-naphthyl)-ethylamine, 1-(2-naphthyl)-ethylamine, 1-phenylpropylamine and desoxyephedrine.

7. A process for obtaining an enantiomer of 3-(2-thienylthio) butyric acid by means of resolving an enantiomeric mixture of 3-(2-thienylthio) butyric acid, comprising:
a) forming diastereoisomeric salts by means of the reaction of enantiomers of said enantiomeric mixture with a resolution agent, wherein said resolution agent is an optically active base;
b) recrystallizing the diastereoisomeric salts obtained in step a) in a crystallization solvent to obtain a diastereoisomeric salt enriched with the desired enantiomer; and
c) obtaining the desired enantiomer by means of:
c.1) resuspending the diastereoisomeric salt obtained in step b) in a suitable medium and rupture of the salt in basic medium.
c.2) neutralizing the separated acid by means of its acidification until achieving a pH comprised between 5 and 1; and
c.3) isolating the desired enantiomer of 3-(2-thienylthio) butyric acid.

8. The process according to claim 7, wherein said optically active base is selected from D-(+)-α-methylbenzylamine, 4-nitrophenyl-methylamine, L-lysine, 1-(1-naphthyl)-ethylamine, 1-(2-naphthyl)-ethylamine, 1-phenylpropylamine and desoxyephedrine.

9. The process according to claim 7, wherein said crystallization solvent is selected from an alcohol, an ester, a ketone, a nitrile, their mixtures, and their mixtures with water.

10. The process according to any of claims 7 to 9, wherein the obtained enantiomer is selected from (3S)-3-(2-thienylthio) butyric acid and (3R)-3-(2-thienylthio) butyric acid.

11. A process for obtaining (33)-3-(2-thienylthio) butyric acid comprising the chiral resolution of an enantiomeric mixture of 3-(2-thienylthio) butyric acid using an optically active base as a resolution agent, in a medium comprising an organic solvent or an aqueous organic mixture as solvent, wherein the diastereoisomeric salts formed are subjected to one or more recrystallizations followed by isolation and recovery of (3S)-3-(2-thienylthio) butyric acid by rupture of the salt in basic medium, phase separation, acidulation of the aqueous phase in the presence of an organic phase and subsequent phase separation.

12. The process according to claim 11, wherein said optically active base is selected from D-(+)-α-methylbenzylamine, 4-nitrophenyl-methylamine, L-lysine, R-1-(1-naphthyl)-ethylamine, R-1-(2-naphthyl)-ethylamine, 1-phenylpropylamine and desoxyephedrine.

13. A diastereoisomeric salt formed by an enantiomer of 3-(2-thienylthio) butyric acid and an optically active base selected from D-(+)-α-methylbenzylamine, 4-nitrophenyl-methylamine, L-lysine, 1-(1-naphthyl)-ethylamine, 1-(2-naphthyl)-ethylamine, 1-phenylpropylamine and desoxyephedrine.

14. (3S)-3-(2-thienylthio) butyrate of D-(+)-methylbenzylamine.

15. A process for the synthesis of (4S,6S)-4-ethylamine-5,6-dihydro-6-methyl-4H-thieno[2,3-b]thiopyran-2-sulfonamide-7,7-dioxide (dorzolamide) comprising carrying out a process according to any of claims 1 to 4, or a process according to any of claims 5 to 7, or the use of the diastereoisomeric salts formed by the reaction of 3-(2-thienylthio) butyric acid with an optically active base according to any of claims 16 or 17 for obtaining the (3S)-3-(2-thienylthio) butyric acid intermediate.
